# EUROPEAN PATENT APPLICATION

(11) **EP 3 547 320 A2**
(43) Date of publication of application: **02.10.2019**
(21) Application number: 17798417.6
(22) Date of filing: 19.05.2017
(51) Int. Cl.: G16C 60/00

(54) **RELATED SYSTEMS AND METHOD FOR CORRELATING MEDICAL DATA AND DIAGNOSTIC AND HEALTH TREATMENT FOLLOW-UP CONDITIONS OF PATIENTS MONITORED IN REAL-TIME**

(30) Priority: 20.05.2016 BR 102016115256
(71) Applicant: Pulse Participações S.A., CEP: 22610-002 Rio de Janeiro RJ (BR)
(72) Inventor: NOBRE, Gustavo de Freitas, CEP-22610002 Rio de Janeiro RJ (BR); KALICHSZTEIN, Marcelo, CEP-22610002 Rio de Janeiro RJ (BR); RAMOS, Marcelo Martinez, CEP-22610002 Rio de Janeiro RJ (BR)
(74) Representative: Isern Patentes y Marcas S.L.
(86) International application number: PCT/BR2017/000049
(87) International publication number: WO 2017/197476

(57) **Abstract**

The present invention describes a system and related methods for analyzing medical data and diagnostic conditions of the patient being monitored at the bedside in real time for purposes of monitoring their health treatment. The system consists of a structured database for storing medical data and examinations on each patient on a central server; integrated data communication modules, which intelligently manage such data, conduct health care planning, and display patient charts and medical reports; and an application server, which interfaces between the database and the communication modules. The methods related to the communication modules of the system refer to the automated perception of health care protocols, correlating medical and examination data to categories of classified diseases, diagnoses and therapeutic procedures, to generate a set of measures to be taken in the prevention and medical conduct. The present invention enables to reduce operational costs and ensure the safety of the medical and patient staff and the improvement of the quality of care and medical treatment. The system has good usability and ease of integration with other systems, equipment and medical devices and can be employed in medical centers, hospitals and residential medical treatment.

## Description

### FIELD OF THE INVENTION

The present invention relates to the technical field of computing and data communication for analyzing medical data and diagnostic conditions of the patient being monitored at the bedside in real time for the purpose of monitoring their health treatment. More particularly, the system consists of: a structured database for storing medical data and examinations on each patient on a central server; integrated data communication modules, which intelligently manage such data, conduct health care planning, and display each patient charts and medical reports; and an application server, which interfaces between the database and the communication modules. The methods related to the communication modules of the system refer to the automated perception of health care protocols, correlating medical and examination data to categories of classified diseases, diagnoses and therapeutic procedures, to generate a set of measures to be taken in the prevention and medical conduct. The present invention enables the result of analyzing patient health information to reduce operational costs and ensure the safety of the medical and patient staff and the improvement of quality of care and medical treatment.

### BACKGROUND OF THE INVENTION

The current health market consumes at least 9 percent of GDP in developed nations, such as England and Canada, whose health systems are considered more efficient, and up to 17 percent of GDP in the US, where waste is estimated to cause injury in the order of up to 20% of consumption. In Brazil, health spending is around 450 billion reais, representing 9.2% of GDP, and the waste is even greater. The per capita expenditure is R $ 2,200.00 in the private system and almost R $ 1,000.00 in the public system. In particular, half the expenses of the operators are directed to hospitalization, including in the occupation of beds in the ICU, which is not well monitored and requires a great effort of work of the medical team. The control of these expenses can therefore have a great impact if these deficiencies that exist today can be increasingly solved with the use of intelligent and automated systems that perform error prevention and waste and that have a care focus.

The concepts of automation have been incorporated for a long time in the medical field, also being used in hospital automation. The concepts of automation have been incorporated for a long time in the medical field, also being used in hospital automation. Hospitals come, over time, computerizing their processes through information systems that perform some tasks pertinent to the hospital environment. Most of these systems are aimed at management and therefore seek to reduce costs and optimize administrative processes. Usually hospitals make use of some of the following systems: electronic medical records; appointment of consultation; pharmacy control; hospitalization; laboratory tests; among others.

The most well-known models of medical information systems are electronic medical records, such as EMR (Electronic Medical Records) or EHR (Electronic Hospitals Records). Although they reduce the costs of a hospital unit, around 20 to 25%, they still leave a more adequate evaluation of the patient's health for the right medical care, since they were born of the administrative need of the hospitals and clinics. They are models that generate bulky medical records, with repeated and many irrelevant data, and are not focused on the patient's specific needs. Another problem is that most models of medical information systems do not automate the collection of patient medical data, which are read and annotated by the nursing staff at the bedside. Therefore, medical data become disaggregated, existing in isolation and at a particular point in time. In other words, if a physician is not actively following certain medical equipment (eg, vital signs monitor of an electrocardiograph) at any given point in time, important data, such as a peak heart rate, may be lost.

Additionally, it is emphasized that human nature makes it difficult for a physician to remember all the items of a clinical protocol or therapeutic procedure guideline, and he ends up not following the whole procedure in a manner consistent with medical practices. Added to this is the fact that despite the diversity of medical data that can be collected from a patient, if these data are not presented in aggregate form, medical information systems end up not producing accurate diagnoses and adequate medical decisions. Most of the time, the available medical data is not coordinated with the patient's medical condition. Thus, they do not mitigate the possibility of error, which is the greatest impact factor in terms of operating costs and process efficiency.

Intelligent information systems that raise the level of patient health care are still underdeveloped. Many of these are evidence-based systems that process query-response methods for retrieving information from previous sets of similar diagnoses. Some process medical data obtained from a patient's electronic records or medical records and indication conditions extracted from clinical protocols and therapeutic guidelines, generating a correlation between this information. Other systems process physiological data from monitors (eg, ECG, EEG), extracting numerical data and basic and complex features of these numerical data, to generate hypotheses related to a patient's medical condition. owever, these systems do not have mechanisms to integrate data communication to automate health care protocols, coordinate such procedures with the patient's medical data, and perform the complete planning of patient evolution to raise the level of health care, as well as to reduce hospitalization costs for hospitals and health care providers.

The present invention proposes, in an unprecedented way, a real-time system that can be accessed locally and remotely and brings together integrated communication modules that perform the following procedures: correlation of medical data and test results of a patient monitored in the bedside with clinical protocols and medical guidelines based on existing terminologies and diseases classified; scaling of the medical team for each patient monitored; medical team decision making about the patient; treatment evolution and patient discharge prediction; available beds in a hospital based on the time of hospitalization / discharge; and visualization of all process information through graphs and reports.

### STATE OF THE ART

The intelligence of medical information systems represents systems, methods, or processes that automate patient health information and medical procedures, increase reliability of results, reduce conversion errors and data analysis, and reduce multiple data acquisition, thus helping to assess the patient's condition and health decision making. US2015 / 0142701 discloses an evidence-based medical record, referring to a system comprising a computer, a computer program and a method for providing an estimate based on confidence estimation. The method includes receiving a question about a patient from a user; accessing an EHR for a patient, wherein the EHR includes a first patient-related component; ask the user, using a conversation interface, for a second component related to the patient, which is based on NLP (natural language program); receiving the second component related to the patient in response to the question; calculating a first probabilistic density function using the first component, and a second function using the second component; combine the first function and the second probabilistic density function using a Gaussian mixed model; compute at least one conditional probability table using the Gaussian model; provide an inference based on confidence estimation based on at least one conditional probability table, the inference being a diagnostic data or medical prognosis.

WO2014 / 126657 refers to latent semantic analysis for application in a question-answer system, effectively improving the punctuation of the response obtained in the medical context through the unification of medical language (concepts and relations). It comprises a system and method that improves the achievement of similarity measurements between concepts based on latent semantic analysis, considering graph structure derived from knowledge bases, using a vector propagation algorithm. The concepts contained in a body of documents are expressed in a graph, where each node represents a concept and the ends between the node represent the relationship between concepts weighted by the number of semantic relationships determined from the body. A vector of neighbors is created and assigned to each concept, providing an improved measure of similarity between documents. The entire process runs on a scheduled processing device.

US2014 / 0244299 discloses a method and device for processing medical data. The method crosses a patient's relevant medical data from an electronic medical record (e.g. EMR) with a plurality of indication conditions obtained from clinical guidelines directed to different diseases, processing predetermined indication conditions related to medical parameters and forming conditional segments based on the respective values of those defined parameters, related to the predetermined indication conditions. The conditional segments correspond to combinations of ranges of values of the medical parameters. The method for processing patient data comprises obtaining patient information distribution information on the plurality of conditional segments and determining a matched relationship of that plurality of patient data with at least one indication condition. The relationship of correspondence is directly determined on the distribution of the patient's data in respective conditional segments, improving the processing efficiency of the patient's data. The device performing such methods includes a processor coupled in communication with a memory.

US2014 / 046890 discloses the analysis, in real time, of hypotheses of ranges of a physiological data using textual representations. Refers to a system in which a physiological data, which comprises numerical data and medical symptoms of a patient, is received in a computer and the processor automatically extracts basic and complex characteristics of said numerical data, which are based on the development of this data to the over a period of time, and automatically converts those features into a textual representation based on NLP. The input terms for an information retrieval system operating on a computer are automatically generated based on those characteristics and represent input into the information retrieval system. A data body is automatically searched to retrieve results related to the input terms, using the information retrieval system.

In US2013 / 0218593, use of designated treatment in medical decision support systems, a method is performed wherein a device receives a multiplicity of medical cases associated with a disease, each case comprising designated medical features and treatment, and the medical cases are divided into at least two groups, each associated with a treatment assigned to medical cases classified within the group. Then, the multiplicity of medical cases divided between two or more groups is used to determine the information, referring to a likely suggested treatment for a sick patient.

US2013 / 0185231 discloses a system and method of predicting a patient's diagnosis, comprising modeling data from a group of successfully diagnosed patients, used as a route of treatment, including references to medical practices; and the diagnostic prediction, which compares a route of treatment of a patient to modeled treatment routes of successfully diagnosed patients, including calculating the probability of a given diagnosis from the modeled treatment routes. The diagnosis can be generated from a single medical condition or from the combination of two or more medical conditions. Automated or manual grouping techniques are used and an example Markov model is used for each possible diagnosis. The probability of each example for each diagnosis is calculated by selecting the diagnostic model example that maximizes the probability of the treatment route.

In US2012 / 0316891, directed cohort selection of a course of medical treatment refers to a method and system for creating a recommended medical treatment course for a patient. A current medical diagnosis of a medical condition suffered by a patient is used to identify a cohort of other persons who have been diagnosed with the same medical condition as the current patient. The set of prior medical procedures used in the cohort members is ordered according to the proximity of the medical treatments, based on the relationship of matching between results and constraints passed on to cohort members and desired outcomes and constraints for the current patient. The sets of ordered medical treatments are presented to the health care provider as a possible course of medical treatment recommended for the current patient.

Patent document PI0715627-8 relates to a method and medical evaluation support system, based on retrieval of information in databases, in which a user enters a query identifying an adverse event and diseases (diseases, disorders , symptoms, conditions, etc.) that a particular patient experienced. In response, the system processes one or more searches to identify one or more possible causes of the adverse event for the patient with the identified diseases. The user may also come in with a combination of one or more drugs that a patient has taken and one or more illnesses that the patient has suffered. The system operates to determine if there is an adverse event associated with the specified combination and reports any adverse events to the user. The system retains a copy of any report for comparison with subsequent searches so as to avoid reporting the same adverse event multiple times. The system performs any search in a predetermined program or can do it by user request. The system integrates "adverse drug - disease" associations with electronic medical record (EMR) systems to identify patients who may present a potential risk in these adverse events and inform health providers or users.

The prior art therefore provides systems and methods for supporting medical evaluation and decision making, assisting medical conduct and patient health treatment. These are systems based on medical evidence that retrieve information from previous medical treatments and diagnoses that correspond to symptoms and illnesses suffered by the current patient. Methods that identify numerical data, range of values or parameters over a period of time associated with clinical guidelines for diagnosing the patient or prior medical evidence are particularly described in US2014 / 0244299 and US2014 / 046890. In the first document, the method differs because the correlation between the medical data and the diagnosis is directly determined on the distribution of the patient's data in segments of respective conditions for each diagnosis, generating statistical results obtained by counting this distribution. In the second document, the method differs in converting the characteristics of a given physician into a textual representation (using natural language) and the system retrieves information based on the characteristics of the medical data through the question-answer system.

In contrast, the present invention proposes a system in integrated data communication modules for carrying out an analysis of medical data and examinations and then monitoring the patient's health treatment. The novelty of the present invention lies in the system of integrated data communication modules and related methods, in particular the correlation between medical data and patient examination results and medical diagnostic conditions. Medical data does not come from electronic medical records, but from real-time monitoring of the patient's vital signs from sensors, devices, and medical equipment installed in the patient, bed, and inpatient setting. The correlation between data and diagnostic conditions occurs by the calculation of automatic digital scores obtained from the medical data in measured numbers, over a period of time, in an automated way at the bedside, from the numerical data of laboratory tests and of ranges of values found in the protocols for each type of symptom, disease or disorder. The results of the digital scores are stored in the evolution of each patient to process other calculations: complexity of the medical staff, better scaling of the medical staff for each patient, prediction of discharge of each patient and balance of beds available for new patients.

The present invention solves the problem of electronic medical records storing excess of disaggregated data from a context more focused on the patient; of the manual collection of medical data, since the data come from the monitoring of the patient's signals, from the bed of the hospitalization environment in real time; of medical conduct, due to the automation and correlation of medical data and clinical protocols; and the patient's complete and adequate follow-up, thus reducing the error through a more precise medical evaluation, reducing the operational costs for hospitals and health care providers and contributing to increase the efficiency of the health treatment process.

### DETAILED DESCRIPTION OF THE INVENTION

The description of the invention makes reference to the following drawings:

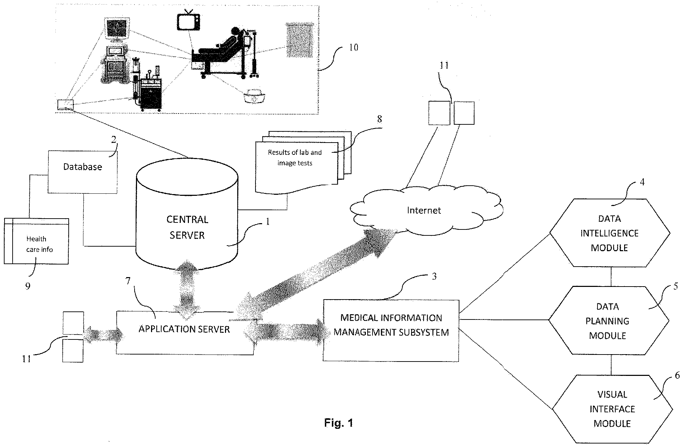

- Drawing 1 shows the block diagram of the system object of the present invention and its operation, wherein (1) represents a central server, containing a database (2), and automatically receives from (8) laboratory test results and image and bed (10) a plurality of medical data of a particular monitored patient. The medical data from (10) and tests from (8) and health care information (9) are stored in the database (2) of (1) and accessed by the health management subsystem (3). (4), a data planning module (5), and a visual interface module (6). An application server (7) interfaces between (1), (3) and its modules (4), (5) and (6) and local and remote users (11

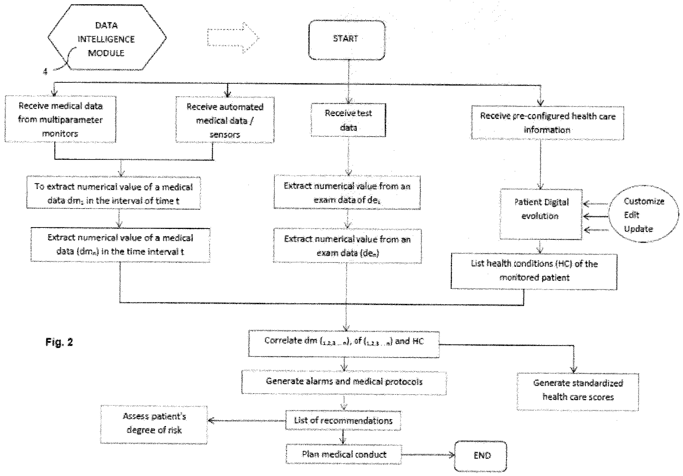

- Drawing 2 details the method of correlation between medical data, examination data and the patient's digital evolution, obtained from pre-configured health care information, to generate alarms and protocols, recommendations and medical conduct, which is performed by the module (4) of the subsystem (3).

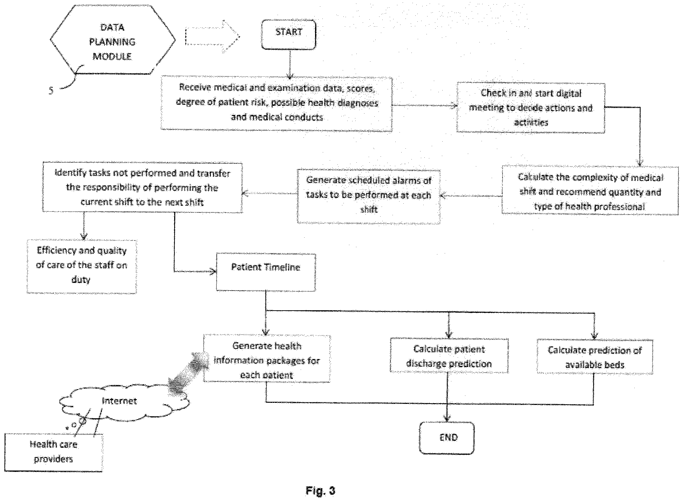

- Drawing 3 details the patient's digital therapeutic planning method, which is performed by module (5) of subsystem (3).

The system consists of integrated data communication modules that receive and evaluate medical data, laboratory and imaging data and patient evolution information being monitored at the bedside in real time. Such information refers to the recording of the conditions of each monitored patient, obtained from a set of preventive measures and preconfigured medical guidelines that are registered in the system for the purpose of monitoring their health treatment. The system consists of a central server (1) containing a database (2), which is structured to store medical and examination data on each monitored patient and, additionally, health care information (9); a medical information management subsystem (3) contains integrated data communication modules (4), (5) and (6), which intelligently manage medical and examination data in conjunction with the digital evolution of the patient. patient and perform health care planning and display each patient's medical charts and reports; and an application server (7) which interfaces between (1), integrated communication modules (4), (5) and (6) of (3) and local and remote users (11).

(1) automatically receives laboratory and imaging results of (8) and a plurality of medical data from the multiparameter and bed automation monitors (10), the signals of which are captured and collected by medical sensors, equipment and devices installed in the monitored patient, at the bedside and in the inpatient setting, and then processed, filtered and interpreted by an intelligence embedded in microprocessors whose system and methods for performing real-time reading and correlation of signals and data processing are the object of the patent application BR10 2016 010619-2. In (2), the data of exams from (8) and the medical data from (10) of the monitored patient are stored, as well as health care information (9) preconfigured to be registered in the system, being accessed in real time by the medical information management subsystem (3) containing modules (4), (5) and (6). The referred data and information are submitted to a medical analysis intelligence and are managed for the purpose of monitoring the patient's medical treatment, with the final results obtained from this intelligence being visualized through graphs and reports. An application server (7) interfaces between (1), (3) and its modules (4), (5) and (6), and local and remote users (11), which can access the results obtained by means of fixed or portable devices such as computers and touch screen devices (smartphones and tablets). Local users refer to the professionals of the care team (doctors and nursing) and the remote users can be these professionals themselves, or even the health care providers who have access to the health information packages of each monitored patient, in order to map authorized and unauthorized services.

The integrated data communication modules of (3) are: data intelligence module (4), data planning module (5) and visual interface module (6). (4) performs the correlation of the medical data of (10), the data of the exams of (8) and the digital evolution of the patient from the automatic perception of the pre-configured health care information (9). The digital evolution of the patient refers to the health conditions of the monitored patient and can be customized, edited and updated by the care team, allowing manually adding other medical information about the patient that is considered relevant. The correlation of these, after being processed, can generate intelligent alarms that attend to the possibility of pathologies or tasks not performed by the care team, besides medical protocols that should be analyzed in the recommendations, prescriptions and medical conduct. From the list of recommendations generated for the care team is also calculated the degree of risk of the patient. The information (9) is registered in the system using existing terminologies and classifications, such as TUSS (Unified Health Terminology) and ICD (International Classification of Diseases). By means of (4) standardized care scores are also generated for the pathologies customized in the system, which are automatic digital scores obtained from the values of the medical data, measured over a period of time, in an automated way in the bed (10), the valuesof the exam data received from (8) and the health conditions of the monitored patient.

Module (4) processes a method that correlates (10), (8) and the digital evolution of the patient obtained from (9) and generates alarms and protocols, standardized care scores, list of recommendations, degree of risk of the patient and medical conduct, for purposes of monitoring the health treatment of the monitored patient, performing the procedures of:
a) Receive medical data from multi-parameter monitors;
b) Receive medical automation / sensor data;
c) Extract numerical value of a medical data dm1 to dmn in the interval of time t;
d) Receive data from laboratory and imaging tests;
e) Extract the numerical value of an examination data from 1 to den;
f) Receive pre-configured health care information;
g) To generate the digital evolution of the patient, whose information can be customized, edited, updated and added;
h) List health conditions (Cs) of the monitored patient;
i) Correlate dm (1,2,3 ... n), of (1,2,3 ... n) and Cs;
j) Generate standardized care scores for customized pathologies in the system;
k) Generate alarms that alert to the possibility of such customized pathologies and other tasks not performed by the care team;
l) Generate medical protocols to be analyzed together with the recommendations of prescriptions;
m) To generate list of recommendations of prescriptions for each professional of the assistance team;
n) Evaluate the degree of risk of the patient;

Examples of non-limiting embodiments of the invention would be the Protocols for Prevention of Mechanical Ventilation Associated Pneumonia (PAV), Pressure Ulcer Prevention (UPP), Prophylaxis for Venous Thromboembolism, Sepsis, Delirium, Diuresis Control, Risk and Fall Prevention, and Attention to Clinical Instability.

The module (5) performs the digital therapeutic planning of the patient, comprising the procedures of:
a) Receive data, scores, indicators and possible diagnoses, obtained in (4), for each patient monitored;
b) Perform check-in by the medical staff on duty, each professional in front of a fixed or portable touch screen equipment, to initiate a digital meeting of those professionals involved in the treatment of a monitored patient, to decide in a group the actions to be carried out and the activities to be performed during the next shift - a procedure called the Multidisciplinary Round;
c) To calculate the complexity of each shift, according to the degree of patient risk obtained in (4), to recommend the best dimensioning of the medical team (quantity and type of health professional) for each patient monitored;
d) Generate scheduled alarms of tasks to be performed by the medical team, according to each patient monitored;
e) Identify tasks performed and not performed on a shift to establish a digital process of transferring responsibility of each patient monitored and signaling the tasks not performed for the next shift - procedure called Digital Change of Shift (DCS);
f) Measure the efficiency and quality of care of the medical team according to the execution of (d) and (e);
g) Generate the timeline of each monitored patient, based on their possible diagnosis, their degree of risk, the scores they received, their medical and examination data, and the main recommendations to be made for allow to automate the whole evolution process of the patient;
h) Gather the timeline information of each monitored patient (possible diagnosis, degree of risk, scores, medical and examination data) to create patient health information packages;
i) Generate health information packages from each monitored patient to the health care providers, in order to map the coverage of services authorized or not for each patient;
j) Carry out occupational provision of beds, according to the patient's degree of risk and the scores received by him, to calculate the prediction of the patient's hospitalization time or prediction, and then the prediction of available beds for new patients - procedure called Discharge Digital Prediction (DDP);

The visual interface module (5) displays the summary of the main occurrences of a dashboard for each patient monitored, in a customized way, in order to visualize processes, tasks and charts and reports, and also displays the timeline of the monitored patient's treatment. All information can be accessed by local and remote users (11), allowing the easy exchange of information via the Internet, including the access of health care providers to each patient's health information packages. The system user can change, update and transmit each monitored patient information and medical decisions in real time.

The system and methods for correlating medical data with diagnostic and monitoring conditions of the real-time monitored patient health treatment object of the present invention can communicate with any medical equipment and devices and standardize the exchange of the data that will be accessed by users. It has good usability and ease of integration with a diversity of systems, equipment and medical devices. The present invention has industrial application, and can be employed in medical centers, hospitals and homecare, allowing the complete follow-up of the patient and the more thorough control of the process, resulting in greater safety of operation of the medical and treatment staff reduction of operating costs.

## Claims

1. A correlation system between medical data and diagnostic conditions and monitoring of the health treatment of a monitored patient in real time, characterized that it comprises:
a) A central server (1) containing a database (2) structured to receive and store medical data from (10) and from (8) from each monitored patient and pre-configured health care information (9)
b) A medical information management subsystem (3) containing integrated data communication modules, comprising a data intelligence module (4), a data scheduling module (5) and a visual interface module (6), to analyze and manage medical and examination data and health care information, conduct health care planning for each monitored patient, and display charts and reports with medical data, scores, indicators, health diagnoses, and medical procedures for each patient monitored;
c) an application server 7, which interfaces between (1), the communication modules (4), (5) and (6) of (3) and the local and remote users (11).

2. A system according to claim 1, **characterized in that** (1) automatically receives laboratory and image examination results from (8) and a plurality of medical data from the multiparameter monitors and the automation of the bed sensors (10) of each monitored patient; (2) storing the medical and examination data and pre-configured health care information (9) which are recorded in (3); (3) and their integrated communication modules (4), (5) and (6) allow analyzing and managing said data and information; complete automated monitoring of the health care of each monitored patient; and display charts and reports with medical data and indicators; (7) to interface between (1), (3) and its modules (4), (5) and (6), and local and remote users (11), allowing said results contained in (3) to be accessed in (11) by means of fixed or portable equipment, comprising fixed computers, portable computers, portable cellular devices and tablets that use touch screen.

3. A system according to claims 1 and 2, characterized that the data intelligence module (4) automatically recognizes the pre-configured health care information (9); to perform a correlation between the data of (10) and (8) and the monitored patient's health conditions obtained from (9) to generate standardized care scores, alarms, protocols and medical recommendations; assess the patient's degree of risk; and plan medical conduct.

4. A system according to claim 3, characterized that the health conditions of the monitored patient are customizable and editable to generate the evolution of the patient's health treatment, so that the processing obtained from (8), (9) and (10) occurs by extracting and correlating the numerical values of the data and the customizable and editable information, calculated in (4) and stored in (2) for each patient.

5. A system according to claim 3, **characterized in that** the preconfigured health care information (9) consists of clinical protocols and therapeutic guidelines in health that use terminologies and existing classifications, comprising TUSS (Unified Health Supplementary Terminology) and ICD (International Classification of Diseases).

6. System according to claims 3 and 4, characterized that the generated alarms allow the identification of possible pathologies or tasks not performed and the protocols generated help to analyze the recommendations and the medical conduct.

7. ystem according to claims 1 and 2, **characterized by** the data planning module (5) performing the activities of: digital meeting of the medical staff to decide actions and activities of the shift; calculating the complexity of medical care; digital shift change; patient timeline; prediction of digital patient discharge; generation of patient health information packages, which allows the complete automated monitoring of the health treatment of each patient monitored and the management of medical information for hospitals and health care providers.

8. System according to claim 7, **characterized by** the digital meeting of the medical team occurring by the confirmation of each professional of this team in (3), using a fixed or portable equipment with touch screen function, to decide on group the actions to be performed in the current shift and the activities to be performed during the next shift.

9. A system according to claim 7, characterized that the complexity of the medical care results in the quantity and type of healthcare professional for each patient.

10. A system according to claim 7, **characterized by** the shift of duty to identify performed and not performed tasks of the current shift for each monitored patient and to signal the tasks not performed for the next shift.

11. A system according to claim 7, **characterized by** a timeline is generated for each monitored patient and consists of its evolution in the health treatment, and (3) it is possible to customize and manually add information about the patient.

12. A system according to claim 7, **characterized by** the digital discharge prediction of each monitored patient calculating the prediction of the patient's hospitalization time and the prediction of available beds for new patients.

13. A system according to claim 7, **characterized by** the health information packets of each patient allow the mapping of authorized and unauthorized services for each patient and the sending to the health care providers.

14. System according to claims 1 and 2, **characterized by** the visual interface module (6) displays the timeline and summary of the main occurrences of the hospitalization in a panel for each monitored patient and allows to visualize processes, tasks and indicators medical and patient evolution management, with customized charts and reports.

15. A system according to claim 14, **characterized by** that (6) allows (3) to be accessed by local and remote users (11), comprising accessing the health information packets of each patient monitored by health care providers.

16. A method of correlating medical data with real-time monitored patient diagnosis conditions, **characterized by** (4) performing the following steps:
a) Receive medical data from multi-parameter monitors;
b) Receive medical automation / sensor data;
c) Extract numerical value of a medical data dm1 to dmn in the interval of time t;
d) Receive data from laboratory and imaging tests;
e) Extract the numerical value of an examination data from 1 to den;
f) Receive pre-configured health care information;
g) To generate the digital evolution of the patient, whose information can be customized, edited, updated and added;
h) List health conditions (Cs) of the monitored patient;
i) Correlate dm (1,2,3 ... n), of (1,2,3 ... n) and Cs;
j) Generate standardized care scores for customized pathologies in the system;
k) Generate alarms that alert to the possibility of such customized pathologies and other tasks not performed by the care team;
l) Generate medical protocols to be analyzed together with the recommendations of prescriptions;
m) To generate list of recommendations of prescriptions for each professional of the assistance team;
n) Evaluate the degree of risk of the patient;
o) Plan medical conduct.

17. A method according to claim 16, **characterized by** that it is applied in the treatment of health and in the prevention of diseases, comprising Prevention of Mechanical Ventilation-Associated Pneumonia (PAV), Pressure Ulcer Prevention (UPP), Diuresis Control, Risk and Prevention of Falls and Assistance to Clinical Instability.

18. A method for monitoring the health treatment of a monitored patient in real time, **characterized by** (5) performing the following steps:
a) Receive medical and examination data, scores, degree of risk, health diagnoses and medical conducts, obtained in (4), for each monitored patient
b) Confirm (3) by the medical staff on shift, each professional in front of a fixed or portable equipment with screen touch, to initiate a digital meeting and jointly decide actions and activities;
c) Calculate the complexity of each medical appointment and recommend the quantity and type of health professional for each patient;
d) Generate task alarms to be performed by the medical team for each patient at each shift;
e) Identify tasks not performed from one shift and transfer the responsibility of execution to the next shift for each patient;
f) Measure the efficiency and quality of care of the medical team according to the execution of (e);
g) Generate the timeline of each patient;
h) Generate health information packages for each patient;
i) Calculate each patient's discharge forecast;
j) Calculate the prediction of available beds for new patients.

19. A method for monitoring the health treatment of a monitored patient in real time according to claim 18, **characterized in that** the complexity of each medical appointment is calculated on the basis of the degree of patient risk obtained in (4).

20. A method for monitoring the health treatment of a monitored patient in real time according to claim 18, **characterized in that** the time line and health information packets of each monitored patient are generated on the basis of medical and examination data, scores, degree of risk, health diagnoses and medical conducts obtained in (4).

21. A method for monitoring the health treatment of a monitored patient in real time, according to claims 18 and 19, **characterized in that** the health information packets are sent to health care providers allowing mapping of the coverage of the authorized services or not for each patient monitored.

22. A method for monitoring the health treatment of a monitored patient in a real-time manner according to claim 18, **characterized in that** the discharge forecast of each monitored patient is calculated based on the patient's time line to perform the provisioning of his / her hospitalization time.

23. A method for monitoring the health treatment of a monitored patient in real time according to claim 18, **characterized in that** the prediction of available beds for new patients is calculated on the basis of the patient's timeline and in his discharge forecast to perform occupational provision of beds.

24. System and methods for correlating medical data and conditions for diagnosis and monitoring of the health treatment of a monitored patient in real time, according to claims 1, 2, 16 and 18, **characterized by** having usability and ease of integration with a diversity of medical systems, equipment and devices.

25. System and methods for correlating medical data and conditions for diagnosis and monitoring of the treatment of a monitored patient's health in real time, according to claims 1, 2, 16 and 18, **characterized in that** they are employed in medical centers, hospitals and in the residential medical treatment, allowing the complete follow-up of the patient and the control of the process, resulting in more safety of operation of the medical team and treatment of the patient and reducing operational costs.
